# EUROPEAN PATENT APPLICATION

(11) **EP 2 650 309 A1**
(43) Date of publication of application: **16.10.2013**
(21) Application number: 11839201.8
(22) Date of filing: 14.05.2011
(51) Int. Cl.: C07K 16/30, A61K 39/395, A61K 47/48, A61K 47/42, A61P 35/00, A61P 37/02

(54) **CONJUGATE OF FOLATE AND ANTIBODY, PREPARATION METHOD AND USE THEREOF**

(30) Priority: 08.11.2010 CN 201010534357
(71) Applicant: Wuhan Huayao Biopharmaceutical Co., Ltd, Wuhan, Hubei 430072 (CN)
(72) Inventor: LIU, Ying, Hubei 430072 (CN)
(74) Representative: Krauss, Jan
(86) International application number: PCT/CN2011/074070
(87) International publication number: WO 2012/062094

(57) **Abstract**

Anti-tumor conjugates, which consists of folic acid or analogues thereof, linkers, an antibodies such as immunoglobulin G. The linker comprises glutathione, cysteamine or cysteine residue, and further comprises N-hydroxysuccinimide. The Conjugates target folate-receptor-positive tumor cells. Also provided are preparation methods and anti-tumor and anti-autoimmune disease uses of the conjugates.

## Description

### TECHNICAL FIELD

The present invention relates to biopharmaceuticals, and specifically to preparation and use of folate-antibody conjugates for the treatment of tumors and autoimmune diseases.

### BACKGROUND OF THE INVENTION

Folic acid was first isolated from spinach leaves by H.K.Mitchell in 1941. It is a water-soluble B-group vitamin composed of pterine, p-aminobenzoic acid and a glutamic acid residue, with a molecular formula of C₁₉H₁₉N₇O₆ and a structural formula of:

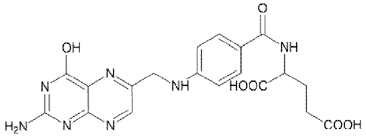

Folic acid is essential for humans and promotes the maturation of cells in the bone marrow. Folate deficiency leads to increase in immature red cells and reduction of white blood cells. Latest studies show that folic acid may protect against cancer through inducing gene expression changes and apoptosis of cancer cell. Importantly, folate binds to the folate receptor. Folate receptor has two membrane isoforms, α and β. Previous reports showed that folate receptor α was highly expressed in 90% of ovarian cancer, as well as at high frequencies in breast cancer, cervical cancer, endometrial cancer, colon cancer, lung cancer, choroidal cancer and ependymoma. Folate receptor β was highly expressed in malignant myeloid cells (leukemia) and in activated macrophages associated with autoimmune diseases such as rheumatoid arthritis. On the contrary, there was almost no expression of folate receptor in normal tissues. Therefore, folic acid and folate receptor have very good potential in the development of targeted therapeutics, especially for the treatment of cancer and autoimmune diseases (Hilgenbrink et al. (2005). J. Pharmaceut. Sci. 94(10): 2135-2146; Lu et al. (2002). Adv. Drug Delivery Reviews 54(5): 675-693. ; Leamon et al., (2001). Drug Discovery Today 6(1): 44-51. ; Lu ct al. (2003). J. Controlled Release 91(1-2):17-29.).

***Current cancer therapies***

The WHO estimated that cancer would overtake heart disease to become the world's most lethal disease in 2010. Compared to 1990, the morbidity and mortality rates of cancer increased by 19% and 18%, respectively. Global cancer rates will increase by 50% by 2020. Each year 15 million new cases will be reported worldwide. Lung cancer is the most common cancer worldwide with 1.2 million new cases each year. The second most common is breast cancer with 1 million new cases each year, followed by colon cancer (0.94 million), gastric cancer (0.87 million), liver cancer (0.56 million), cervical cancer (0.47 million) and esophageal cancer (0.41 million). The global prevalence of cancer is increasing, constituting a serious threat to human life.

Current treatments for cancer include: surgery, chemotherapy, radiation therapy and immunotherapy. These treatments are often combined. Due to inevitable side effects of chemotherapeutics, the development of new drugs is necessary. Recently, the drugs based on monoclonal antibodies are favored by the cancer patients, such as Herceptin™, Avastin™ and Rituxan™. Compared to conventional chemotherapy drugs, the biggest advantage of these drugs is high efficiency and low toxicity (Adams et al., (2005). Nature Biotechnology 23(9): 1147-1157; Reichert et al., (2007). Nature Reviews Drug Discovery 6(5): 349-356.).

The current market for antibody drugs is estimated at US$12 billion/year, but their costs of development are also very high. They also have many disadvantages such as high quality control requirements, immunogenicity, and off-target effects (Kuus-Reichel et al. (1994), Clinical and Vaccine Immunology 1(4): 365; Ito et al. (1992), Cancer Research 52(7): 1961).

***Current therapies for autoimmune diseases***

An autoimmune disease is a condition that occurs when the immune system mistakenly attacks and destroys healthy tissues. Clinically, autoimmune disease is often characterized by a serum gamma globulin, IgG content of >1.5%; presence of autoimmune antibodies in affected organs; deposition of denatured gamma globulin or corresponding antigen in the glomeruli; infiltration of a large number of lymphocytes and plasma cells in the lesion. Application of adrenal cortical hormone can obtain temporary or continuous relief.

Autoimmune diseases include systemic lupus, erythematosus, rheumatoid arthritis, inflammatory bowel disease and psoriasis. Despite many years of research, these diseases cannot be completely cured.

Drugs commonly used for autoimmune diseases include nonsteroidal anti-inflammatory drugs (NSAIDs) such as aminosalicylic acid, indomethacin, propionic acid derivatives and antacid drugs, for incipient and mild diseases. The mechanisms are inhibiting COX enzymes and production of prostaglandins to achieve anti-inflammatory analgesic effect. But they cannot prevent the progression of rheumatoid arthritis. In addition, because of overlaping metabolic pathways, interactions among these drugs may occur and combined application is not recommended.

Gold salts such as gold sodium thiomalate is also used. Other drugs include penicillamine, chloroquine, levamisolem, immunosuppressant drugs, and adrenal cortical hormone, each of which has their own shortcomings.

As previously mentioned, folate receptor α is highly expressed in multiple types of cancer cells, and folate receptor β is highly expressed in white blood cells of myeloid leukemia or activated macrophages of some autoimmune diseases such as rheumatoid arthritis, yet there is almost no expression of folate receptor in most normal tissues. Accordingly, radiolabeled folic acid conjugates (such as conjugates of folic acid and ¹²⁵I, ⁶⁷Ga and ¹¹¹In) have been used to detect tumor tissues with high expression of folate receptors. Also, folic acid-protein toxin, folic acid-small molecule chemotherapy drugs, folic acid-liposomes (Liposomes containing chemotherapy drugs or gene-based drugs) and folic acid-immunotherapeutic agents are being developed as anti-cancer agents. (see e.g. Hilgenbrink et al. (2005). J. Pharmaceutical Sciences 94(10): 2135-2146; Pan et al. (2002). Bioconjugate Chem 13(3): 435-442; Li et al. Bioconjugate Chemistry, 2010, 21(5): 961-968; Hassan, R., W. Ebel, et al. (2007), Cancer Immun 7: 20).

### DESCRIPTION OF THE INVENTION

In light of the deficiencies of the existing technology, the present invention provides a high-efficacy folate-conjugated antibody (folate-GSH-IgG), including its preparation methods and use in treating cancer and autoimmune diseases.

A first objective of the present invention is to provide a high-efficacy folate-GSH-IgG conjugate, which is characterized by the coupling of an activated IgG with folate through GSH. The molecular formula of GSH is C₁₀H₁₇N₃O₆S. The structure of this conjugate is shown in formulae I, II and III below:

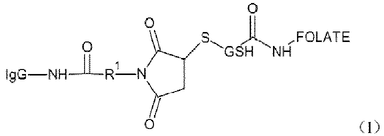

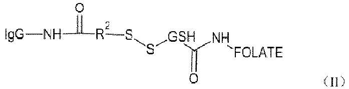

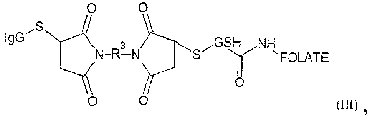

wherein FOLATE represents folate or a folate derivative such as methotrexate, tetrahydrofolate, dihydrofolate, the structural formula is as follows:

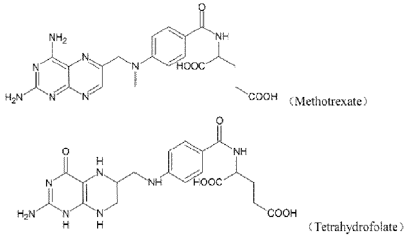

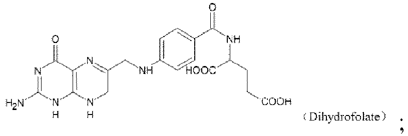

GSH represents glutathione with a molecular formula of C₁₀H₁₇N₃O₆ S, or a chemical compound with a free sulfhydryl and a free amino group, such as cysteamine and cysteine, and structural formulae of:

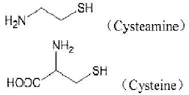

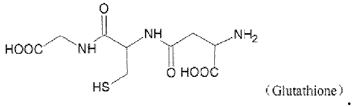

IgG may be an animal IgG, human IgG, recombinant humanized IgG, a half-IgG, a Fc fragment, or a heavy chain thereof from a reduction reaction. In formula III, IgG represents the above IgG with an active sulfliydryl.

Any one of the four subtypes of IgG: IgG1, IgG2, IgG3 and IgG4 can be used. IgG may be reduced to a half-IgG by a weak reducing agent such as 2-mercaptoethylamine · HCl, and it also can be reduced to IgG heavy chains by a strong reducing agent like 1,4-dithiothreitol (DTT):

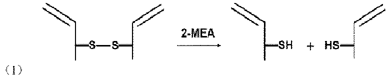

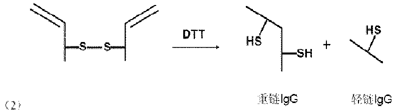

In formula I, R¹ is selected from the groups in Table 1.

**Table1**

| R1 | Formula |
|---|---|
| AMAS | -CH₂- |
| BMPS | -CH₂CH₂- |
| EMCS , Sulfo-EMCS | -CH₂CH₂CH₂CH₂CH₂- |
| GMBS, Sulfo-GMBS | -CH₂CH₂CH₂- |
| LC-SMCC | 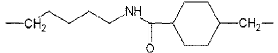 |
| MBS , Sulfo-MBS | 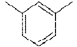 |
| SMCC , Sulfo-SMCC | 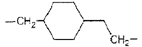 |
| SMPB , Sulfo-SMPB | 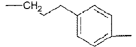 |
| SM(PEG)n NHS-PEG-Maleimide Crosslinkers | 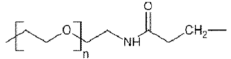 |
| SMPH | 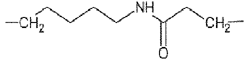 |
| Sulfo-KMUS | 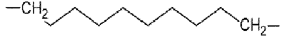 |

In formula II, R2 is selected from the groups in Table 2.

**Table2**

| R2 | Formula |
|---|---|
| LC-SPDP, Sulfo-LC-SPDP | 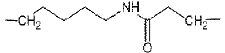 |
| SMPT | 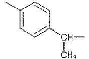 |
| SPDP | -CH₂CH₂- |
| Sulfo-LC-SMPT | 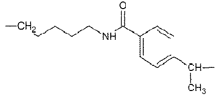 |

In formula III, R³ is selected from the groups in Table3.

**Table 3**

| R3 | Formula |
|---|---|
| BM(PEG)2 | 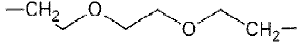 |
| BM(PEG)n PEG Crosslinkers | 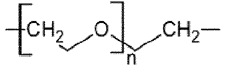 |
| BMB | -CH₂CH₂CH₂CH₂- |
| BMDB | 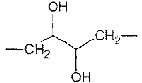 |
| BMH | -CH₂CH₂CH₂CH₂CH₂CH₂- |
| BMOE | -CH₂CH₂- |
| DPDPB | 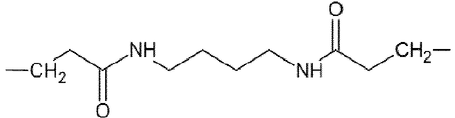 |
| DTME | -CH₂CH₂SSCH₂CH₂- |
| TMEA | 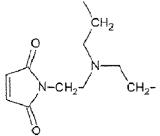 |

After activation by DCC and NHS, folate is linked with GSH through an amide bond to form folate-GSH. Then, the amino terminus (N-terminal) or the epsilon amino groups of lysine residues of IgG is activated to produce an activated form of IgG. IgG also can be reduced to half-IgG or heavy chain. Both of these two types of IgG could be coupled with folate-GSH.

In the first method, the IgG amino group is activated. The activation agent mentioned may have a structure of RₐCOR_{b}, wherein Rₐ is a leaving group as a part of an amine-reactive function such as N-hydroxysuccinimide, whose formula is shown in formula IV. R_{b} contains a functional group reactive to the free sulfhydryl in GSH, preferably a maleimide, as shown in formula V:

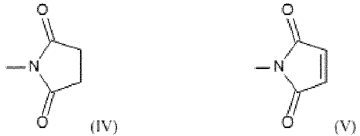

The activation agents are selected from Sulfo-LC-SPDP (sulfosuccinimidyl 6-[3' (2-pyridyldithio)-propionamido] hexanoate), LC-SPDP (succinimidyl 6-[3' (2-pyridyldithio)-propionamido] hexanoate), SPDP (N-succinimidyl-3-(2-pyridyldithio) propionate), SMPT (4-Succinimidyloxycarbonyl-methyl-alpha-[2-pyridyldithio]toluene), Sulfo-LC-SMPT (4-Sulfosuccinimidyl-6-methyl-alpha-(2-pyridyldithio) toluamido] hexanoate)), Sulfo-SMCC (Sulfosuccinimidyl 4-[N-maleimidomethyl] cyclohexane-1-carboxylate), SM(PEG)n NHS-PEG-Maleimide Crosslinkers, SMCC (Succinimidyl-4-[N-maleimidomethyl] cyclohexane-1-carboxylate), LC-SMCC (Succinimidyl-4-[N-Maleimidomethyl] cyclohexane-1-carboxy-[6-amidocaproate]), Sulfo-EMCS ([N-e-Malcimidocaproyloxy]sulfosuccinimide ester), EMCS ([N-e-Maleimidocaproyloxy] succinimide ester), Sulfo-GMBS (N-[gamma-Maleimidobutyryloxy] sulfosuccinimide ester), GMBS (N-[gamma-Maleimidobutyryloxy] succinimide ester), Sulfo-KMUS (N-[k-Maleimidoundecanoyloxy] sulfosuccinimide ester), Sulfo-MBS (m-Maleimidobenzoyl-N-hydroxysulfosuccinimide ester), MBS (m-Maleimidobenzoyl-N-hydroxysuccinimide ester), Sulfo-SMPB (Sulfosuccinimidyl 4-[p-maleimidophenyl] butyrate), SMPB (Succinimidyl-4-[p-maleimidophenyl] butyrate), AMAS N-(alpha-Maleimidoacetoxy) succinimide ester) BMPS (N-[ ß -Maleimidopropyloxy] succinimide ester) SMPH (Succinimidyl-6-[ ß -maleimidopropionamido]hexanoate) or other groups that can react with free sulfhydryl.

The products after IgG reacts with Sulfo-SMCC, SM(PEG)n NHS-PEG-Maleimide Crosslinkers, SMCC, LC-SMCC, Sulfo-EMCS, EMCS, Sulfo-GMBS, GMBS, Sulfo-KMUS, Sulfo-MBS, MBS, Sulfo-SMPB, SMPB, AMAS, BMPS and SMPH have the common structure of formula VI.

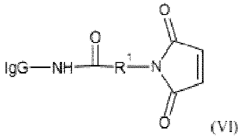

wherein R1 represents a group listed in Table 1.

Folate-GSH-IgG conjugate is formed after reacting folate-GSH with the activated IgG of formula VI. This conjugate has the structure shown in formula I.
After IgG reacts with Sulfo-LC-SPDP, LC-SPDP, SPDP, SMPT and Sulfo-LC-SMPT, the products have the common structure of Formula VII:

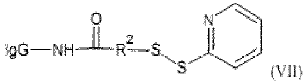

wherein R2 represents a group listed in Table 2.

Folate-GSH-IgG conjugate is formed after reacting folate-GSH with the activated IgG of formula VI. This conjugate has the structure shown in Formula II.

If IgG mentioned above is a half-IgG or heavy chain with reactive sulfhydryl, it could also be activated through sulfhydryl-reactive linkers such as 1,8-bis-Maleimidodiethyleneglycol (BM(PEG)2), BM(PEG)n PEG Crosslinkers, 1,4-bismaleimidobutane (BMB), 1,4 bismaleimidyl-2,3-dihydroxybutane (BMDB), bismaleimidohexane (BMH), Bis-Maleimidoethane (BMOE), 1,4-Di-[3'- (2 -pyridyldithio)-propionamido]butane (DPDPB), Dithio-bismaleimidoethane (DTME) and Tris[2-maleimidoethyl]amine (TMEA) or their derivatives. These compounds have common structure shown in formula VIII. R3 represents a group listed in Table 3.

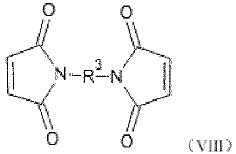

During the formation of folate-GSH-IgG, folate is linked with GSH through an amide bond after activation by DCC and NHS. Free sulfhydryl in the half IgG or heavy chain reacts with products of formula VTTT and yields a compound with the structure of formula IX.

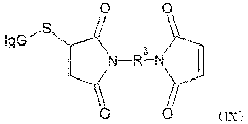

This compound will react with folate-GSH to produce the conjugates of formula III.

For convenience of understanding, a list of conjugates with different structures follows:
1. Folate-GSH-IgG conjugates produced by coupling of folate-GSH and IgG through SPDP; 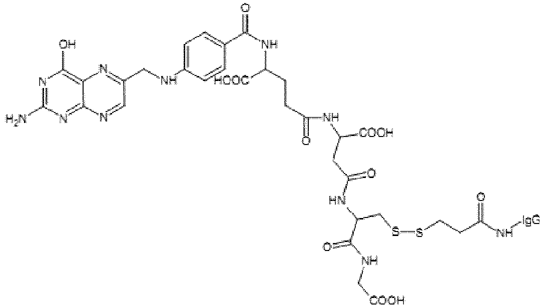 which is simplified as: 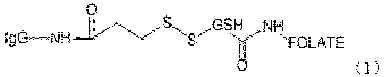
2. Folate-GSH-IgG conjugates produced by coupling of folate-GSH and IgG through SMPB; 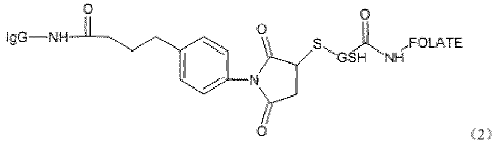
3. Folate-GSH-IgG conjugates produced by coupling of folate-GSH and IgG through SMCC; 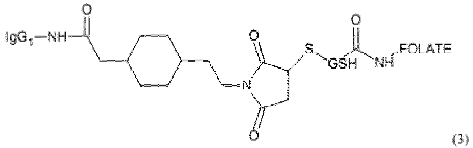
4. Folate-GSH-IgG conjugates produced by coupling of folate-GSH and reduced form IgG (weak reducing agent) through BMB; 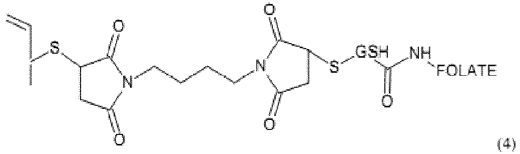
5. Folate-GSH-IgG conjugates produced by coupling of folate-GSH and reduced form IgG (strong reducing agent) through DTME; 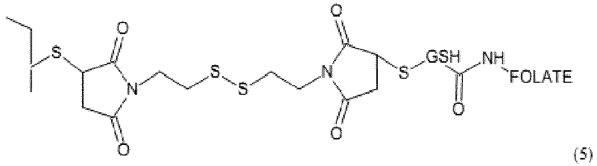
6. Folate-GSH-IgG conjugates produced by coupling of folate-GSH and reduced form IgG (weak reducing agent) through EMCS; 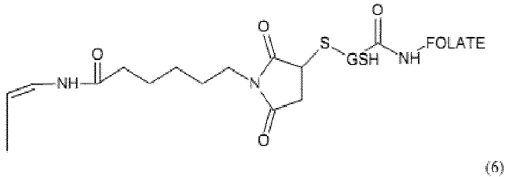
7. Folate-GSH-IgG conjugates produced by coupling of folate-GSH and reduced form of IgG (strong reducing agent)) through LC-SPDP 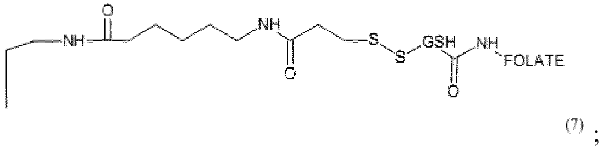
8. Folate-GSH-IgG conjugates produced by coupling of folate-GSH and IgG1 through Sulfo-GMBS; 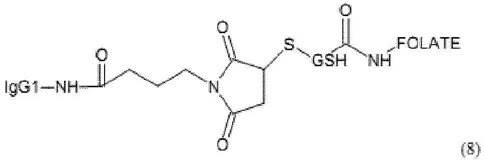
9. Folate-GSH-IgG conjugates produced by coupling of folate-GSH and IgG1 through Sulfo-KMUS; 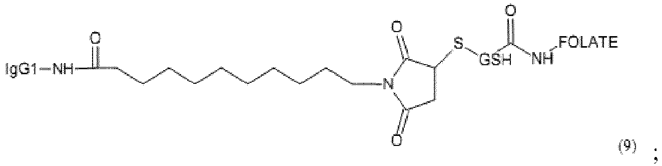 and
10. Folate-GSH-IgG conjugates produced by coupling of Methotrexate - GSH and IgG1 through Sulfo-KMUS;

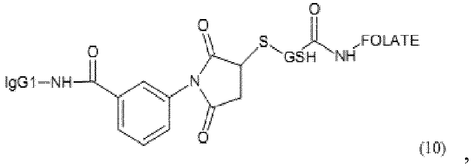

wherein FOLATE in formula 1-9 is folic acid and in formula 10 it is Methotrexate.

Folate-GSH-IgG conjugates provided in the present invention specifically recognize and bind to receptors of target cells with higher expression of folate receptors. These bindings induce the cytotoxic effect through activation of effector cell or complement system mediated by IgG and enhances the function of biological response modifier such as IL-2. Normal cells with lower expression of folate receptors are not affected. Therefore, this invention avoids the problem that traditional drugs damage normal cells in the body.

The present inventors recognized that folate-GSH-IgG conjugates can be used in the treatment of cancer and autoimmune disease, and have shown experimentally that the conjugates have good therapeutic effects. Furthermore, because there is no exogenous protein sequence in the conjugates, there is no antibody neutralization reaction in vivo. Meanwhile, IgG could enhance the effects of the conjugate. The compounds are effective by binding to cell surfaces to activate downstream reactions, without the need for cellular internalization. Further research showed that, the GSH component of the conjugate leads to an increase of free carboxyl content and hydrophilicity of the folate ligand and extends the space between folic acid and IgG leading to significant improvement of the targeting of the folate receptor and treatment efficacy.

In another embodiment, the present invention provides the synthetic methods of this stable conjugate, comprising the steps of:
1. Folate is reacted with N-hydroxysuccinimide to form NHS-folate;
2. NHS-folate is reacted with GSH through an amide bond to form folate-GSH;
3. IgG is activated at its amino groups to form a sulfhydryl reactive form, which has a structure of formula VI-VII or formula IX, respectively;

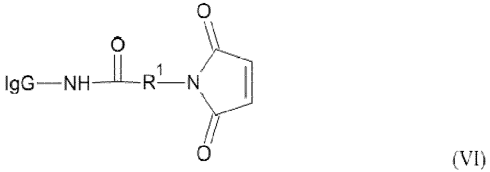

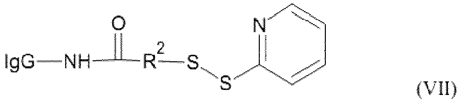

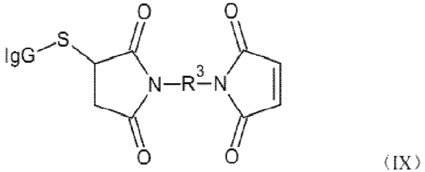

wherein, R¹, R² and R³ as defined above.

The parameter and conditions involved in these steps are further elucidated as follows.
1. Folate is reacted with N-hydroxysuccinimide to form NHS-folate, wherein Folate represents folate or a folate derivative, such as Methotrexate, Tetrahydrofolate, Dihydrofolate, and folic acid is the preferred choice.
   Preferably, folate or folate derivatives are dissolved in DMSO and reacted with N, N' -dicyclohexylcarbodiimide and N-hydroxysuccinimide for 2-5 hr. The insoluble byproducts are discarded through centrifugation or filtration. The soluble constituent contains activated NHS-folate. The reaction equation is shown below with folic acid as an example: 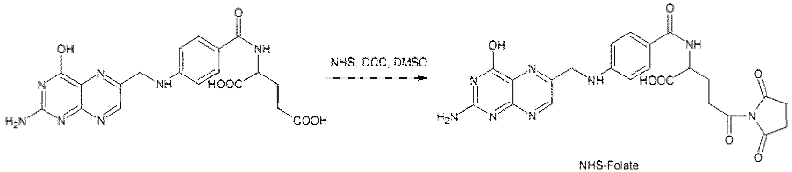
2. Folate-GSH is generated after the reaction between NHS-folate and GSH, which are connected through an amide linkage. Preferably, NHS-folate reacts with an equal amount of GSH overnight in the dark and under argon. The final product has the structure of following formula: 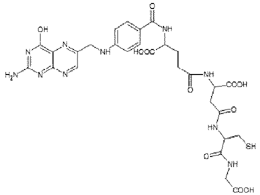
3. Preparation of activated IgG: The IgG amino groups may be activated through an amide bond, or IgG is reduced to half-IgG or heavy chains.

### i. The activation of IgG amino group

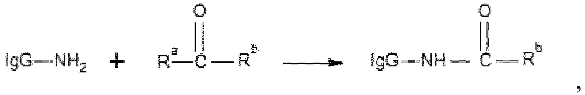

wherein IgG-NH2 represents amino group of IgG. IgG is selected from human IgG, murine IgG, recombinant IgG, the Fc fragments of these IgG and the half-IgG or heavy chain through reduction. In the present invention, IgG is any one of the 4 types of IgG: IgG1, IgG2, IgG3 and IgG4. R^{a}COR^{b} is IgG activator with a leaving group R^{a}. Succinimide of formula IV is preferred. R^{b} is the group responsible for reacting with free sulfhydryl of GSH. Maleimide of formula V is preferred.

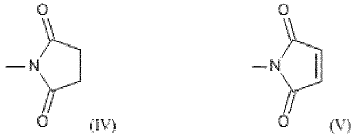

If the activators are Sulfo-SMCC, SM(PEG)n NHS-PEG-Maleimide Crosslinkers, SMCC, LC-SMCC, Sulfo-EMCS, EMCS, Sulfo-GMBS, GMBS, Sulfo-KMUS, Sulfo-MBS, MBS, Sulfo-SMPB, SMPB, AMAS, BMPS and SMPH, the generated compounds have a common structure of formula VI after reaction with IgG. 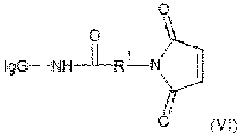 wherein R¹ is defined above.

If the activators are Sulfo-LC-SPDP, LC-SPDP, SPDP, SMPT and Sulfo-LC-SMPT, the generated compounds have a common structure of formula VII after reaction with IgG. 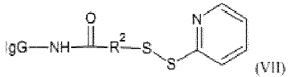 wherein: R² represents a group shown in Table 2.

Preferably IgG is dissolved in a suitable solvent to the concentration of 0.05-1 mmol/L. This solution reacts with 10-30-fold of activators mentioned above for 0.5-1 h at room temperature or 1-3 h at 4□. This activated IgG is purified through a molecular sieve column such as PD-10 (Amersham Biosciences). The suitable solvent can be 10 ∼ 30 mmol/L PBS-EDTA (pH6.5 - 9.0), ultrapure water , DMSO or DMF. 10 - 30 mmol/L PBS-EDTA is the preferred choice.

### ii. Reduction of IgG:

After treatment with the reducing agent, the disulfide bond in the heavy chains, or between the heavy chains and the light chains are separated, exposing the active sulfhydryl.

IgG is reduced to half-IgG with weak reducing agent such as 2-Mercaptoethylamine · HCl (2-MEA). 1 ml 2 ∼ 20 mg/ml IgG-PBS-EDT A buffer (20 - 100 m M Na₃PO₄, 150 mM NaCl, 1 ∼ 10mM EDTA, pH 6.0 ∼ 8.0) is mixed homogeneously with 6 mg 2-MEA or other weak reducing agent for 1-3 h at 37□(If the volume of IgG solution is less than 1 ml, 6 mg 2-MEA can be dissolved in PBS-EDTA and mixed with IgG solution rapidly in a 1000-400:1 proportion). After cooling to room temperature the reaction mixture is purified through a desalting column such as PD-10. The reaction equation is as follows.

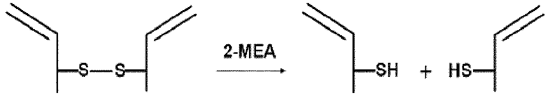

IgG is reduced to heavy chains with a strong reducing agent such as 1, 4-Dithiothreitol (DTT). DTT is dissolved in solution (pH 6.5-9.0) with a final concentration of 0.1-20 Mm. DTT solution is reacted with IgG solution for 1-4 h at room temperature and the mixture is loaded in a desalting column immediately to obtain the heavy chain of IgG. The reaction equation is as follows.

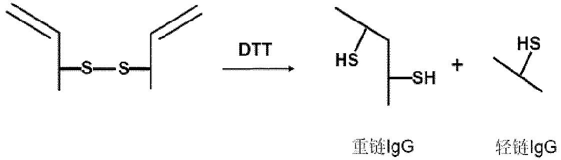

IgG can also be activated by sulfhydryl reactive linkers such as 1,8-bis-Maleimidodiethyleneglycol (BM(PEG)2), BM(PEG)n PEG Crosslinkers, 1,4-bismaleimidobutane (BMB), 1,4 bismaleimidyl-2,3-dihydroxybutane (BMDB), bismaleimidohexane (BMH), Bis-Maleimidoethane (BMOE), 1,4-Di-[3'-(2'-pyridyldithio)-propionamido] butane (DPDPB),

Dithio-bismaleimidoethane (DTME), Tris[2-maleimidoethyl]amine (TMEA) and their analogues, whose common structure is formula VIII, wherein R3 represents a group listed in Table 3. The following conjugate will be generated:

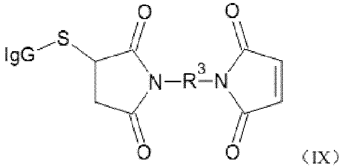

### 4. Folate-GSH is coupled with IgG to yield the folate-GSH-IgG conjugate.

As mentioned above, the activated IgG is coupled with folate -GSH and forms the folate -GSH-IgG conjugate with the structure of formula 1 and 11. The half-IgG or heavy chain activated by sulfhydryl reactive linkers is coupled with folate-GSH and form the folate -GSH-IgG conjugate with the structure of Formula III.

In a preferred embodiment, folate-GSH reacts with 1-10-fold of activated IgG for 2-5 h at room temperature or 15-20 h at 4□. The product is purified and collected with molecular sieve chromatography.

Step 3 could be conducted simultaneously or prior to step 1 and 2.

In another embodiment, the present invention provides a pharmaceutical composition comprising an effective amount of a folate-GSH-IgG conjugate and a pharmaceutically acceptable carrier or excipient. A suitable dosage form may be lyophilized powder or injection solution. The table below lists a specific example of the composition of the present invention.

| **Components** | **Weight ratio** |
|---|---|
| folate-GSH-IgG conjugate | 1 ∼ 20% |
| Maltose | 10 % |
| Glucose (or NaCl) | 5 ∼ 10 % (0.9 %) |
| Vitamin C (or other antioxidants) | 0.01 ∼ 2.0 % |
| Sterile water for injection | Up to 100% |

The present invention also provides for the use of a folate-GSH-IgG conjugate in the preparation of antitumor medicines.

As mentioned above, in patients with cancer or autoimmune diseases, a folate-GSH-IgG conjugate provided here specifically binds the folate receptor on cancer cells or on activated macrophages. Through the IgG, this binding activates antibody or complement system, and/orenhance the function of biological response modifier such as IL-2. The cancer cells will be killed by the resulting cytotoxicity. But there is no effect on normal cells with lower expression of folate receptor. Based on mechanism analysis, as long as the cancer cells express high level of folate receptor, folate-GSH-IgG will be effective with a proper dosage. Cancer types suitable for treatment by the composition of the present invention includes leukemia, cervical cancer, breast cancer, endometrial cancer, colon cancer, lung cancer, liver cancer, choroidal carcinoma and ependymoma. The conjugates can be delivered through injection such as i.v., i.m. or s.c. injection. Suitable dosage ranges for human of 1mg ∼ 4g/kg and 5 mg ∼ 100 mg/kg are preferred.

The present invention further provides for the use of a folate-GSH-IgG conjugate in the preparation of drugs for treating autoimmune diseases.

Owing to the complex pathological features, recurrence and difficulty to cure, autoimmune disease leaves patients in great suffering. A large number of immune cells are activated and cytokines are released during the pathological process. And macrophages play an important role in disease development. Folate-GSH-IgG conjugates provided herein selectively bind the folate receptor in the activated macrophage. In addition, there are the following mechanisms that works in concert with folate receptor targeting. Firstly, the conjugate competitively binds the IgG Fc receptor on macrophages, reducing phagocytosis and secretion of inflammatory factors. Secondly, folate-GSH-IgG effectively inhibits the antigen presentation of macrophages, inhibiting the activation of T lymphocytes, secretion of inflammatory factors and the lethal effect of cytotoxic T-cells. Finally, folate-GSH-IgG blocks the Fc receptor on macrophages, thereby preventing phagocytosis of autologous tissues already bond to autoantibody (e.g. anti-platelet antibody).

Autoimmune diseases mentioned in the present invention are diseases caused by immune responses to self-antigens, which can damage autologous tissues. Accordingly as long as the disease is characterized by a high level of folate receptor expression, folate-GSH-IgG will be effective with a proper dosage. These autoimmune diseases include rheumatoid arthritis, inflammatory bowel disease, psoriasis, lupus erythematosus, pulmonary fibrosis and sarcoidosis. The conjugates can be delivered through injection through the vein, muscle or through subcutaneous injection. The range of prescribed doses is 1mg ∼ 4g/kg body weight and 5 mg ∼ 100 mg/kg is preferred.

Further descriptions are given below by examples. It should be pointed out that in the animal studies, the IgG of folate-GSH-IgG was from mice. This avoids Fc receptor mismatch and immunogenicity of humanized IgG in mice. In the future, when the conjugate is applied in humans, human IgG will be used.

### DESCRIPTION OF THE FIGURE

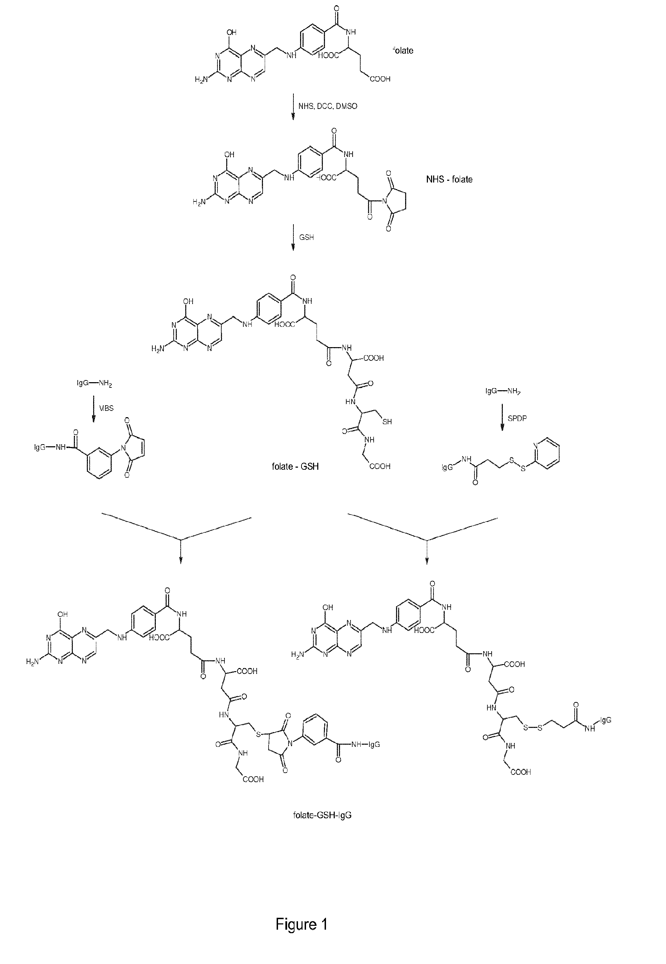

Figure 1 is a reaction diagram to generate folate-GSH-IgG. After activation with DCC and NHS, folic acid is reacted with GSH through an amide bond to form folate-GSH. Simultaneously, amino group in IgG is activated by MBS or SPDP respectively. These two kinds of activated IgG are coupled with folate-GSH to produce different folate-GSH-IgG conjugates.

### Examples

**Materials:** BMB, DCC, DTT, DTME, EMCS, FITC, Folic acid, GSH, LC-SPDP, 2-MEA, Methotrexate, NHS, SMCC SMPB SPDP, Sulfo-GMBS, and Sulfo-KMUS, are purchased from Sigma. Recombinant IgG is from Leinco Technologies. Fc portion is from Sino Biological. Human IgG1 is from AXXORA.

Fetal Bovine Serum and MEM culture medium are provided by Hyclone.

MCF-7 breast cancer cell line is from Wuhan University Culture Collection center.

### Example 1

Folate-GSH-IgG conjugate comprises folate, GSH and IgG. IgG is a recombinant IgG.

A mixture of 0.1mmol folate, 0.1mmol DCC and 0.1mmol NHS is kept in DMSO for 2h at room temperature, followed by centrifugation. To get Folate-GSH, 0.1 mmol GSH is added in the supernatant to be reacted overnight in dark and under argon environments. IgG is mixed with 10-fold of SPDP (from 20mM stock solution) for 0.5 h at room temperature. The mixture is loaded on a molecular sieve column PD-10 to get activated IgG. Folate-GSH reacted with 0.1 equivalent of activated IgG at RT for 15h. The product folate-GSH-IgG is collected through molecular sieve column.

### Example 2

IgG is Fc portion of human IgG.

A mixture of 30 micromol folate, 40 micromol DCC and 30 micromol NHS is kept in DMSO for 5h at RT, followed by centrifugation. To get Folate-GSH, 30 micromol GSH is added in the supernatant to be reacted overnight in dark and Argon environment. IgG is mixed with 30-fold of SMPB (from 20mM stock solution) for 1 h at room temperature. The reaction mixture is loaded on molecular sieve column PD-10 to get activated IgG. Folate-GSH reacts with 20-fold by weight of activated IgG at 4□ for 20 h. The product folate-GSH-IgG is then collected through molecular sieve column PD-10.

### Example 3

IgG is human IgG1.

A mixture of 10 micromol folate, 20 micromol DCC and 10 micromol NHS is kept in DMSO for 3h at RT, followed by centrifugation. To get Folate-GSH, 10 micromol GSH is added in the supernatant to be reacted overnight in dark and Argon environment. IgG is mixed with 20-fold of SMCC (20mM stock solution) for 1 h at room temperature. The reaction mixture is loaded on molecular sieve column PD-10 to get activated IgG. Folate-GSH reacts with 10-fold by weight of activated IgG at RT for 20 h. The product folate-GSH-IgG is then collected through molecular sieve PD-10.

### Example 4

IgG is human IgG1.

At 37□, 6 mg 2-MEA is dissolved in 1ml PBS-EDTA buffer (20 - 100 mM Na₃PO₄, 150 mM NaCl, 1 ∼ 10mM EDTA, pH 6.0 - 8.0) which contains 2 ∼ 20 mg/ml IgG for 1h.

The product, cooled to room temperature, is loaded on a desalting column such as PD-10 to get half-IgG (reduced IgG). The mixture of 10 micromol folate, 20 micromol DCC and 10 micromol NHS is kept in DMSO for 3h at RT, followed by centrifugation. To get Folate-GSH, 10 micromol GSH is added in the supernatant to be reacted overnight in dark and Argon environment. In PBS (pH 7.5), the sulfhydryl-cross-linker BMB dissolved in DMF is reacted with 1-fold reduced IgG at RT for 2 h. The product folate-GSH-IgG is collected through desalting column PD-10.

### Example 5

IgG is human IgG1.

DTT is dissolved in PBS-EDTA (pH 7.5) at 5 mM. The mixture of IgG solution and DTT is kept for 2 h at RT.

The product is loaded on a desalting column such as PD-10 immediately to get IgG heavy chain.

A mixture of 10 micromol folate, 20 micromol DCC and 10 micromol NHS is kept in DMSO for 3h at RT, followed by centrifugation. To get Folate-GSH, 10 micromol GSH is added to the supernatant to be reacted overnight in dark and Argon environment. In PBS (pH 7.5), the sulfhydryl-cross-linker DTME dissolved in DMF is reacted with 1-fold reduced IgG at RT for 2 h. The resulting product is then reacted with folate-GSH. The product folate-GSH-IgG is collected through desalting column PD-10.

### Example 6

IgG is human IgG1.

At 37□, 6 mg 2-MEA is dissolved in 1ml PBS-EDTA buffer (20 - 100 mM Na₃PO₄, 150 mM NaCl, 1 ∼ 10mM EDTA, pH 6.0 - 8.0) which contains 2 ∼ 20 mg/ml IgG for 1h.

The product, cooled to room temperature, is loaded on a desalting column such as PD-10 to get half-IgG.

The mixture of 10 micromol folate, 20 micromol DCC and 10 micromol NHS is kept in DMSO for 3h at RT, followed by centrifugation. To get Folate-GSH, 10 micromol GSH is added in the supernatant to be reacted overnight in dark and Argon environment. At RT, LC-SPDP (20mM stock solution) is used to reduce IgG for 1h. Folate-GSH is then added to 10-fold by weight of the reduced IgG at RT for 20 h. The product folate-GSH-IgG is collected through molecular sieve column PD-10.

### Example 7

IgG is human IgG1.

DTT is dissolved in PBS-EDTA (pH 7.5) at 5 mM. A mixture of IgG solution and DTT is kept for 2 h at RT. The product is loaded on desalting column such as PD-10 to get IgG heavy chain.

The mixture of 10 micromol folate, 20 micromol DCC and 10 micromol NHS is kept in DMSO for 3h at RT, followed by centrifugation. To get Folate-GSH, 10 micromol GSH is added in the supernatant to be reacted overnight in dark and Argon environment. At RT, the IgG heavy chain is reacted to 20-fold LC-SPDP (20mM stock solution) for 1h and separated with PD-10 column. Folate-GSH then reacts with 10-fold by weight of the activated IgG at RT for 20 h. The product folate-GSH-IgG is collected through PD-10 column.

### Example 8

IgG is human IgG1.

The mixture of 10 micromol folate, 20 micromol DCC and 10 micromol NHS is kept in DMSO for 3h at RT, followed by centrifugation. To get Folate-GSH, 10 micromol GSH is added in the supernatant to be reacted overnight in dark and Argon environment. At RT, IgG is activated with 20-fold Sulfo-GMBS (from a 20mM stock solution) for 1h and separated with PD-10 column. Folate-GSH then reacts with 10-fold by weight of activated IgG at RT for 20 h. The product folate-GSH-IgG is collected through PD-10 column.

### Example 9

IgG is human IgG1.

The mixture of 10 micromol folate, 20 micromol DCC and 10 micromol NHS is kept in DMSO for 3h at RT, followed by centrifugation. To get Folate-GSH, 10 micromol GSH is added in the supernatant to be reacted overnight in dark and Argon environment. At RT, IgG is activated by 20-fold Sulfo-KMUS (from a 20mM stock solution) for 1h and separated with PD-10 column. Folate-GSH then reacts with 10-fold by weight of activated IgG at RT for 20 h. The product folate-GSH-IgG is collected through PD-10 column.

### Example 10

Folate analogue-GSH-IgG conjugate comprises methotrexate, GSH and IgG. The molecular formula of methotrexate is C₂₀H₂₂N₈O₅. IgG is human IgG1.

A mixture of 10 micromol Methotrexate, 20 micromol DCC and 10 micromol NHS is kept in DMSO for 3h at RT, followed by centrifugation. To get methotrexate-GSH, 10 micromol GSH is added in the supernatant to be reacted overnight in dark and Argon environment. At RT, IgG is reacted with 20-fold SMCC (from a 20mM stock solution) for 1h and separated with PD-10 column. Methotrexate-GSH reacts with 10-fold activated IgG at RT for 20 h. The product folate-GSH-IgG is collected through PD-10 column.

### Example 11

Comparison of the stability of folate-GSH-IgG prepared by two methods

Experimental materials
1. Folate-GSH-IgG conjugate is prepared according to embodiment 3. Fetal Bovine Serum and MEM culture medium are provided by Hyclone.
2. MCF-7 breast cancer cell line is from Wuhan University Culture Collection center. Methods:

Preparation of folate -IgG conjugate: A mixture of 30 micromol folic acid, 40 micromol DCC and 30 micromol NHS was kept in DMSO for 3h at RT, followed by centrifugation. The supernatant was reacted with 20-fold by weight of recombinant IgG for 5h at RT. The product was purified and collected through molecular sieve PD-10.

Folate-IgG and folate-GSH-IgG were incubated with 5-fold of FITC for 2h at RT, respectively. FITC labeled conjugates, called FIG-FITC and FGIG-FITC, were loaded onto a PD-10 column to be collected. Half of these were kept in dark for 3 months at 4□. Cell fluorescence intensity were detected as follows.

MCF-7 cells (stably transfected with folate receptor) in logarithmic phase were resuspended with MEM containing 10% fetal bovine serum. The single cell suspension was cultured in 96-well culture plate (5 × 10⁴/ml) with 200 µl / well. The cells were divided into several groups: negative control, FIG-FITC, FGIG-FITC, FIG-FITC -3, FGIG-FITC -3 (FIG and FGIG were kept in darkness for 3 months, 4□) and folic acid interference group (Folate+ FIG-FITC, Folate+FGIG-FITC). Each group had 3 replicates. 20 µl of FIG or FGIG-FITC was added in each well. In the folic acid interference group, the folic acid-PBS was put into the wells (the final concentration of folic acid is 20 µmol/L). After culture in 5%CO2 at 37□for 3 h, the samples were washed 3 times with 200 µl PBS. Cells were observed under inverted fluorescence microscopy after digestion with pancreatin. The fluorescence absorbed by cells could also be detected with fluorometer after cell lysis and centrifugation in 1000 rpm for 20 min.

### Results:

FITC labeled folate-IgG and folate-GSH-IgG could bind the folate receptor efficiently to make the tumor cells green. But the interference of folic acid significantly decreased the fluorescence intensity. This indicates the specificity of the binding between tumor cell and folate-IgG or folate-GSH-IgG. Meanwhile, compared with folate-IgG, folate-GSH-IgG effectively improved the uptake of cells (about 45.2%). In addition, the binding experiments also showed a significant difference after the conjugate was kept for 3 months at 4□. In Table 4, the fluorescence intensity of folate-IgG group was weaker than folate-GSH-IgG group, and the uptake of folate-IgG group was just 69.8% of folate-GSH-IgG group. These results suggest that the improved preparation methods strengthen the targeting and stability of folate-GSH-IgG.

**Table 4. Comparisons of uptake in different groups**

| | Group | Uptake (%) |
|---|---|---|
| Immediate | Control | 2.53 ± 0.23 |
| | FIG-FITC | 64.23 ± 12.43 |
| | FIG-FITC+Folate | 34.82 ± 2.43 |
| | FGIG-FITC | 3.23 ± 21.74 ** |
| | FGIG-FITC+Folate | 29.34 ± 3.25 |
| 4°C, 3 months | Control | 4.72 ± 0.38 |
| | FIG-FITC | 58.82 ± 13.12 |
| | FIG-FITC+Folate | 20.93 ± 2.28 |
| | FGIG-FITC | 84.29 ± 18.39 ^{▲▲} |
| | FGIG-FITC+Folate | 24.20 ± 2.49 |

| | | |
|---|---|---|
| **, P<0.01 compared with the on-line results of FIG-FITC group; ▲▲, P<0.01 compared with FIG-FITC group kept in 4°C for 3 months. | | |

**Example 12** Use of folate-GSH-IgG for treating breast cancer

### Experimental materials

1. Folate-GSH-IgG conjugate (FGIG) and folate-IgG (FIG) conjugate are prepared according to embodiment 1 and 11, respectively. IgG is mouse IgG from Sigma.
2. Animal: 50 healthy and mature female nude mice, body weight 18-22 g are provided by Nanjing University Model Animal Center.

Animal grouping and processing

By weight, the mice were randomly divided into normal control group, model group, Epirubicin positive control group, FIG 5mg/kg group and 40 mg/kg group, FGIG 5mg/kg group and 40 mg/kg group. 7 days before the beginning of the experiment, the mice were maintained with feed without folic acid. From day 0, model group, epirubicin positive control group, FIG 5mg/kg group or 40 mg/kg group and FGIG 5mg/kg group or 40 mg/kg group were subcutaneously injected with folate receptor transfected MCF-7 cells (1 × 10⁶) each. From day 5, FIG 5mg/kg group and FIG 40mg/kg group were intra-peritoneally injected with FIG (5mg/kg and 40mg/kg, respectively). FGIG 5mg/kg group and FGIG 40mg/kg group were intra-peritoneally injected with FGIG (5mg/kg and 40mg/kg, respectively). Epirubicin positive control group were intra-peritoneally injected with Epirubicin (200 mg/kg). Normal control group and model group were intra-peritoneally injected equal amount of saline. The injections were performed one time every four days. 15 days after injection, the mice were fasted. The next day after treatment, tumor volume was measured with vernier caliper and mean survival time was also calculated.

Volume: solid tumor volume was calculated using the following formula: V = 0.5×L×W×H.

Results

1. The inhibition by FGIG of MCF-7 cell proliferation in DBA/2 mice

As shown in Table 2, the injection of 5mg/kg and 40mg/kg FGIG effectively inhibit of the proliferation of MGF-7 in DBA/2 mice. After 16 days, the volume of solid tumor in these two groups was reduced 50.8% and 63.3% and smaller than FIG groups, indicating that the inhibition by folate-GSH-IgG on MCF-7 proliferation is greater than folate- IgG.

2. FGIG increased mean survival time of DBA/2 mice

Injection of 5mg/kg and 40mg/kg FGIG increased mean survival time of the mice by 2.0 or 3.0-fold, but there was no significant difference between the folate-GSH-IgG and folate- IgG groups.

**Table 5 The effect of FIG on tumor volume and mean survival time of mice (X±SD, n=10)**

| Group | Volume of tumor (cm³) | Mean survival time (days) |
|---|---|---|
| normal control group | - | 16 |
| model group | 13.95 ± 3.89** | 3** |
| FGIG 5mg/kg | 6.87 ± 1.84 ^{▲▲} | 9 ^{▲▲} |
| FGIG 40mg/kg | 5.12 ± 2.12 ^{▲▲} | 12 ^{▲▲} |
| FIG 5mg/kg | 7.35 ± 1.39 ^{▲▲} | 8 ^{▲▲} |
| FIG 40mg/kg | 6.83 ± 1.94 ^{▲▲} | 9 ^{▲▲} |
| Positive control group | 6.18 ± 1.34 ^{▲▲} | 9 ^{▲▲} |

| | | |
|---|---|---|
| **, P<0.01 compared with normal control group; A A, P<0.01 compared with model group. | | |

**Example 13** In vitro test of FOLATE-GSH-IgG as anti-cancer drugs

FOLATE-GSH-IgG was selected as an anti-cancer drug. The growth inhibitory effects on Human leukemia cell K562, cervical cancer cell Hela, breast cancer cell MCF-7, colon cancer cell Caco-2 and liver cancer cell HepG2 were tested using MTT colorimetric assay. The half inhibitory concentration (IC50) was also measured.

Experimental materials
1. Drugs and reagents: FOLATE-GSH-IgG conjugate (FGIG) and FOLATE-IgG (FIG) conjugate prepared according to embodiment 1 and 11, respectively.
2. Human leukemia cell line K562, cervical cancer cell line Hela, breast cancer cell line MCF-7, colon cancer cell line Caco-2 and liver cancer cell line HepG2 are from Wuhan University Culture Collection center.

Cancer cell inhibition (CI): CI=(OD_{control group} -OD_{experimental group})/OD _{control group} *100%

Inhibitory concentration 50 (IC₅₀): Cancer cell growth rate was plotted with different concentrations of the same samples to obtain a dose-response curve and the IC50 calculated.

Results

FOLATE-GSH-IgG significantly inhibited the proliferation of K562, Hela, MCF-7, Caco-2 and HepG2 cells in a dose dependent way, probably due to cancer cell apoptosis induced by FOLATE-GSH-IgG binding. FOLATE-GSH-IgG had more cytotoxicity than FOLATE-IgG.

**Table 6 IC50 of FOLATE-GSH-IgG**

| Cell line | FOLATE-GSH-IgG (nmol/L) | FOLATE-IgG (nmol/L) |
|---|---|---|
| K562 | 1 .40 ± 0.27 | 2.82 ± 0.21 |
| Hela | 3.32 ± 0.02 | 4.54 ± 0.05 |
| MCF-7 | 5.53 ± 0.32 | 6.12 ± 0.83 |
| Caco-2 | 8.29 ± 3.82 | 9.39 ± 5.29 |
| HepG2 | 8.23 ± 1.39 | 10.39 ± 2.82 |

**Example 14** Folatc-GSH-IgG for treatment of inflammatory bowel diseases

The effects and mechanisms of folate-GSH-IgG for treating inflammatory bowel disease were studied using animal models.

Experimental materials
1. folate-GSH-IgG conjugate (FGIG) and folate-IgG (FIG) conjugate are prepared according to embodiment 1 and 11, respectively. IgG is mouse IgG from Sigma. Mesalazine SR Granules are from Ethypharm (France). MPO detection kit is from Nanjing Jian-Cheng Bioengineering Institute.
2. Animal: 50 healthy and mature male Kunming mice, body weight 18-22 g are provided by Nanjing University model animal center.

Animal grouping and processing

The mice were randomly divided into control group, model group, mesalazine positive control group, FIG 5mg/kg group and 40 mg/kg group, FGIG 5mg/kg group and 40 mg/kg group. Wherein, FIG 5mg/kg group and 40 mg/kg group were i.p. injected with FIG (5mg/kg and 40mg/kg, respectively). FGIG 5mg/kg group and FGIG 40mg/kg group were i.p. injected with FGIG (5mg/kg and 40mg/kg, respectively). Mesalazine positive control group were given 600mg/kg Mesalazine orally Control group and model groups were i.p. injected with equal amount of saline. The injections were performed once per day.

Acetic acid stimulation was used to induce colitis. The mice were fasting on day 9, and on day 10, were anaesthetized with ether inhalation. Before injection, the intestinal was cleaned, with a polyethylene pipe containing 0.1ml 6% acetic acid inserted about 1.5 cm into the anus.

Testing Criteria
1. CMDI is an acronym for "colon mucosa damage index: The entire colon was taken and scissored along the mesenteric. The adhesion, ulcer and congestion were scored by naked eye observation according to the Table 7.
2. Myeloperoxidase (MPO): The tissues were weighted and homogenized (1:19, weight/volume) in Tris buffer. After homogenization and repeated freezing and thawing, the tissues were made to 5% homogenate. MPO detection was performed according to kit manufacturer's instructions and the protein level was detected using the Lowry method.

**Table 7 Colon mucosa damage index**

| Overall Appearance | | Score |
|---|---|---|
| **Abdominal adhesions** | | |
| | Without adhesion | 0 |
| | Mild adhesion | 1 |
| | Severe adhesion | 2 |

| **Colon ulcer and inflammation** | | |
|---|---|---|
| | No obvious abnormity | 0 |
| | Colon congestion, no ulcer | 1 |
| | Congestion, no ulcer, bowel wall thickening | 2 |
| | Ulcer in one place, without congestion or bowel wall thickening (Surface necrosis) | 3 |
| | Intestinal wall bleeding | 4 |
| | One ulcer, with hyperemia inflammation | 5 |
| | Two ulcer, with hyperemia inflammation | 6 |
| | More than two ulcers | 7 |
| | One puncture | 8 |
| | More than one puncture | 9 |
| | Death | 10 |

The colon coefficient is the sum of the 2 scores above.

**Results**

Table 8 shows that folate-GSH-IgG significantly alleviated colon inflammation of mice with inflammatory bowel disease. After injection of 5mg/kg and 40mg/kg folate-GSH-IgG, colon coefficient was reduced 42.0% and 64.8%, respectively. Inflammation score level fell by 1.44 times and 4.93 times. MPO activity decreased 58.3% and 71.3%. These results were better than folate-IgG. Low or high dose of folate-GSH-IgG could obviously protect the mice from colon injury induced by acetic acid. The mice colonic rating level was 3.4 and 1.4, respectively. In model group, there were severe adhesions and more than 2 or more ulcers. The mice colonic rating level was 8.3. There were still mild redness and adhesion. In FIG group, mild irritation still existed but there was no obvious ulcer and adhesion, and the colonic rating level was 1.9.

**Table 8 Effects of folate-GSH-IgG on colon coefficient, inflammation score and activity of MPO in mice with inflammatory bowel disease (X±SD, n=10)**

| Group | Colon coefficient (g/100g) | Inflammation rating level | MPO (U/mg.pro.) |
|---|---|---|---|
| Normal control | 0.72 ±0.34 | 0 ± 0 | 1.24 ± 0.35 |
| Model group | 1.93 ± 0.38** | 8.3 ± 1.3** | 7.49 ± 0.43** |
| FGIG 5mg/kg | 1.12 ± 0.20 ^{▲▲} | 3.4 ± 0.7 ^{▲▲} | 3.12 ± 0.25 ^{▲▲} |
| FGIG 40mg/kg | 0.68 ± 0.21 ^{▲▲} | 1.4 ± 0.3 ^{▲▲} | 2.15 ± 0.33 ^{▲▲} |
| FIG 5mg/kg | 2.39 ± 0.37 ^{▲▲} | 4.9 ± 1.3 ^{▲} | 5.20 ± 1.20 ^{▲} |
| FIG 40mg/kg | 1.20 ± 0.39 ^{▲▲} | 2.7 ± 0.5 ^{▲▲} | 3.29 ± 0.42 ^{▲▲} |
| Positive control group | 1.21 ± 0.13 ^{▲▲} | 1.9 ± 0.5 ^{▲▲} | 2.42 ± 0.11 ^{▲▲} |

| | | | |
|---|---|---|---|
| **, P<0.01 compared to normal _{control} group; ▲▲, P<0.01 compared to model group. | | | |

**Example 15** Use of folate-GSH-IgG for treating of rheumatoid arthritis

The effects and mechanisms of folate-GSH-IgG in treatment of rheumatoid arthritis were studied through rheumatoid arthritis animal models.

Experimental materials
1. Folate-GSH-IgG conjugate (FGIG) and folate-IgG (FIG) conjugate are prepared according to embodiment 1 and 11, respectively Rat IgG and complete Freund adjuvant are from Sigma. Diclofenac sodium enteric-coated tablets are from Beijing Novartis Pharma Ltd. RF detection kit is from Nanjing JianCheng Bioengineering Institute.
2. Animal: 50 healthy and mature Wistar rat, both male and female, body weight 150 ∼ 200g are provided by Wuhan University Laboratory Animal Center, China.

Methods

### ANIMAL GROUPING AND PROCESSING

The rats were randomly divided into normal control group, model group, Diclofenac sodium positive control group, FIG 2mg/kg group and 8 mg/kg group, FGIG 2mg/kg group and 8 mg/kg group. Wherein, FIG 2mg/kg group and 8 mg/kg group were Intra-peritoneally injected with FIG (2mg/kg and 8mg/kg, respectively). FGIG 2mg/kg group and FGIG 8mg/kg group were Intra-peritoneally injected with FGIG (2mg/kg and 8mg/kg, respectively). Diclofenac sodium positive control group were given 10mg/kg Diclofenac sodium orally. Normal control group and model group were Intra-peritoneally injected equal amount of saline. The injections were performed once per day. After measurement of right foot volume on day 0, 0.1ml complete Freund adjuvant was subcutaneously injected into right ankle. On day 21, the right foot volume was measured after the last injection. Rats were sacrificed with ether inhalation after taking blood samples. The blood samples were centrifuged to get serum for the detection of rheumatoid factor (RF).

Testing criteria
1. Joint injury index score: Table 9
2. **Rheumatoid** factors RF: 100 µl serum were used with RF detection kit.

**Table 9 Joint injury index score standard**

| **Overall Appearance** | **Score** |
|---|---|
| No apparent abnormality | 0 |
| Mild redness in toe joints | 1 |
| Moderate redness in toe joints | 2 |
| Severe redness in toe joints | 3 |
| Contralateral redness in toe joints | 4 |

**Results**

In Table 10, folate-GSH-IgG could significantly alleviate toe redness of rats with rheumatoid arthritis. After injection of 2mg/kg and 8 mg/kg folate-GSH-IgG, Digit or toe volume difference was decreased by 70.9% and 78.8%, respectively. Joint injury score level decreased 1.58 and 5.20 times. The content of rheumatoid factors decreased by 19.1% and 25.8%, respectively These results were better than folate-IgG. Compared with 8 mg/kg folate-IgG group, the joint injury score level and toe volume difference of rats in 8 mg/kg folate-GSH-IgG group decreased by 54.5% and 36.9%. These show that intraperitoneal injection of folate-GSH-IgG efficiently protects the rats from rheumatoid arthritis.

**Table 10 Effects of folate-GSH-IgG on joint injury score, toe volume difference and activity of RF in rats with rheumatoid arthritis (X±SD, n=10)**

| Group | Joint injury score | Toe volume difference (ml) | RF (pg/ml) |
|---|---|---|---|
| Normal control | 0.0 ± 0.0 | 0.052 ± 0.022 | 14.3 ± 0.73 |
| Model group | 3.1 ± 0.21** | 0.419 ± 0.089* | 22.5 ± 1.23** |
| FGIG 2mg/kg | 1.2 ± 0.11 ^{▲} | 0.122 ± 0.112 ^{▲} | 18.2 ± 1.16 ^{▲} |
| FGIG 8mg/k | 0.5 ± 0.36 ^{▲▲} | 0.089 ± 0.063 ^{▲▲} | 16.7 ± 1.97 ^{▲▲} |
| FIG 2mg/kg | 1.7 ± 0.21 ^{▲} | 0.187 ± 0.125 ^{▲} | 20.2 ± 2.14 |
| FIG 8mg/kg | 1.1 ± 0.57 ^{▲▲} | 0.141 ± 0.103 ^{▲} | 19.7 ± 1.39 ^{▲▲} |
| Positive control | 0.6 ± 0.22 ^{▲▲} | 0.090 ± 0.052 ^{▲▲} | 15.2 ± 1.99 ^{▲▲} |

| | | | |
|---|---|---|---|
| *P<0.05, **P<0.01 compared to normal control group; A, P<0.05, A A, P<0.01 compared with model group. | | | |

**Example 16** Use of folate-GSH-IgG to treat lupus erythematosus

Experimental materials:

1. Drugs and reagents: folate-GSH-IgG conjugate (FGIG) and folate-IgG (FIG) conjugate are prepared according to embodiment 1 and 11, respectively. Mouse IgG and complete Freund adjuvant are from Sigma. HRP-goat anti mouse IgG is from Ministry of Health, Beijing Institute of Biological Products. IL-1 and RF detection kits arc from Nanjing JianCheng Bioengineering Institute.

2. Animal: 50 health and mature female Kunming mice, body weight 18-22 g are provided by Wuhan university model animal center. The production license is: (SCXK (Hubei) 2003-2004). Mouse feed is purchased from Wuhan university laboratory animal center.

Methods

Animal grouping and processing

The mice were randomly divided into normal control group, model group, Tripterygium Wilfordii positive control group, FIG 1mg/kg group and 10 mg/kg group, FGIG 1mg/kg group and 10 mg/kg group. FIG 1mg/kg group and 10 mg/kg group were celiac injected with FIG (1mg/kg and 10mg/kg, respectively). FGIG 1mg/kg group and FGIG 10mg/kggroup were celiac injected with FGIG (1mg/kg and 10mg/kg, respectively). Tripterygium Wilfordii positive control group were given 10mg/kg tripterygium wilfordii orally. Normal control group and model group were celiac injected equal amount of saline. The injections were performed once per day.

Suspension of formaldehyde Campylobacter jejuni (3 × 10¹²CFU/L) was mixed with complete Freund adjuvant equally. After completely emulsification, 50 µl of suspension was injected into foot metatarsus. 3 weeks later, 10 µl of suspension was injected again by intravenous injection. 28 days after sensitization, the autoantibody of histone in serum was detected.

Testing Criteria

Autoantibody of histone: the serum at 1:100 dilution was applied to the coated 96-well plate. The samples were incubated for 2h at 37 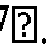 . Then added Horseradish peroxidase -coupled goat anti-mouse IgG (1:100) and incubated for 2h at 37 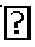 . The substrate was added and incubated for 20min at 37. The reaction was terminated with 2 mol/L H₂SO₄. Absorbance score was recorded at 490 nm and this was shown with enzyme index (EI).

Results

In Table 11, folate-GSH-IgG could significantly reduce the production of histone autoantibody of mice with lupus erythematosus. After injection of 1mg/kg and 10mg/kg folate-GSH-IgG, production of histone autoantibody was reduced 38.7% and 47.1%, respectively. The high dose group had notable effects compared with positive control group. In the 10 mg/kg group, folate-GSH-IgG decreased the production of histone autoantibodyby 12.5% compared to folate-IgG. These show that folate-GSH-IgG efficiently decreases the production of histone autoantibody, better than folate -IgG.

**Table 11 Effects of folate-GSH-IgG on autoantibody of histone in mice with lupus erythematosus (X±SD, n=10)**

| Group | Autoantibody of histone (EI) |
|---|---|
| Normal control | 53 ± 12 |
| Model group | 119 ± 31** |
| FGIG 1mg/kg | 73 ± 20 ^{▲▲} |
| FGIG 10mg/k | 63 ± 13 ^{▲▲} |
| FIG 1mg/kg | 85 ± 32 ^{▲} |
| FIG 10mg/kg | 72 ± 21 ^{▲▲} |
| Positive control | 60 ± 11 ^{▲▲} |

| | |
|---|---|
| ** P<0.01 compared with normal control group; A P<0.05, ▲▲ P<0.01 compared with model group. | |

**Example 17** Folate-GSH-IgG for the treatment of psoriasis

The effects and mechanisms of folate-GSH-IgG in treatment of psoriasis were studied through animal models.

Experimental materials
1. Drugs and reagents: folate-GSH-IgG conjugate (FGIG) and folate-IgG (FIG) conjugate are prepared according to embodiment land 11, respectively. Mouse IgG is from Sigma. Triamcinolone acetonide ointment is from Tianjin pharmaceutical company.
2. Animal: 40 health and mature Kunming mice, body weight 18-22 g, male and female half-and-half are provided by Wuhan University model animal center. The production license is: (SCXK (Hubei) 2003-2004). Mouse feed is purchased from Wuhan university laboratory animal center.

Methods

Animal grouping and processing

Mice were randomly divided into normal control group, model group, triamcinolone acetonide ointment positive control group, FIG 1mg/kg group and 10 mg/kg group, FGIG 1mg/kg group and 10 mg/kg group. Wherein, FIG 1mg/kg group and 10 mg/kg group were celiac injected with FIG (1mg/kg and 10mg/kg, respectively). FGIG 1mg/kg group and FGIG 10mg/kg group were celiac injected with FGIG (1mg/kg and 10mg/kg, respectively). Triamcinolone acetonide ointment positive control group were given 0.05g triamcinolone acetonide ointment in the root of the tail. Normal control group and model group were celiac injected equal amount of saline. The injections were performed once per day. After continuous administration for 15 days the mice were sacrificed. A strip of tail skin 1.8 cm far from tail root was taken and fixed with formaldehyde solution (100 ml/ L). After paraffin embedding and HE staining, the mouse-tail scale epidermis was observed under optical microscope.

Testing Criteria

Number of scales of the mouse-tail epidermis with granular layer: Scales with lined granular layer between two hair follicles are classified as scales wtih granular layer. The number of scales wtih granular layer per 100 scales is measured and compared among different groups.

Results

In Table 12, folate-GSH-IgG could significantly increased the production of scales with granular layer. After injection of 1mg/kg and 10mg/kg folate-GSH-IgG, the number of scales of granular layer increased 1.64 and 2.21 times, respectively.

**Table 12 Effects of folate-GSH-IgG on scales with granular layer in the tail epidermis of mice with psoriasis (X±SD, n=10)**

| Group | Number of scales wtih granular layer |
|---|---|
| Normal control | 10.3 ± 3.44 |
| FGIG 1mg/kg | 27.2 ± 5.12** |
| FGIG 10mg/k | 3.1 ± 11.21** |
| FIG 1mg/kg | 18.8 ± 3.29* |
| FIG 10mg/kg | 29.4 ± 8.30** |
| Positive control | 28.5 ± 6.86** |

| | |
|---|---|
| ** P<0.01 compared with normal control group. | |

## Claims

1. A conjugate of Formula (I), (II) or (III), 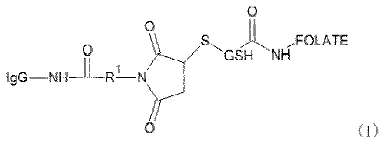 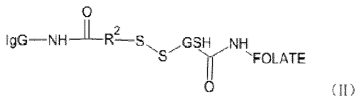 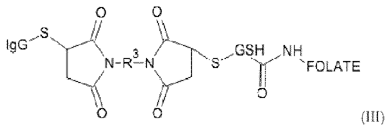 wherein:
FOLATE is folate or a folate analogue that binds a cell surface folate receptor, including methotrexate, tetrahydrofolate and dihydrofolate,
GSH is glutathione or a compound comprising both a free sulfhydryl and a free amino group, or Cysteamine or Cysteine,
IgG is immunoglobulin G or a monomer of an IgG heavy chain or a Fc fragment thereof from reduction of IgG, an animal IgG, a human IgG, or a recombinant humanized IgG,
R¹ is 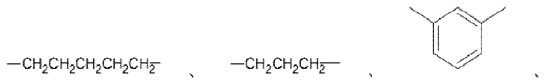 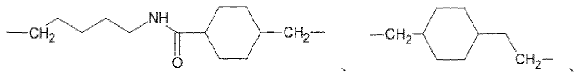 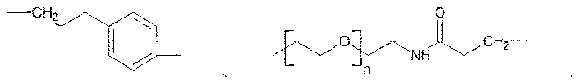 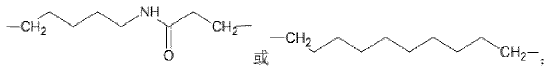
R² is 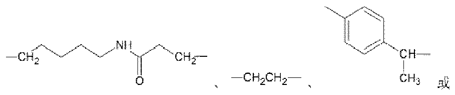 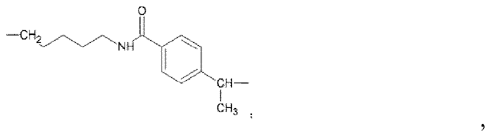
and R³ is

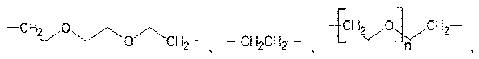

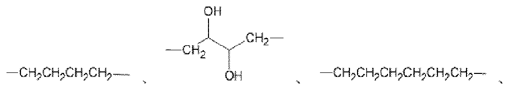

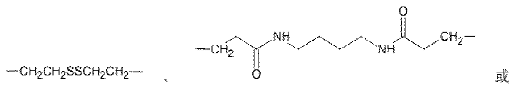

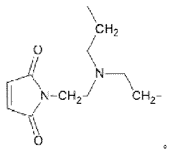

2. The conjugate of claim 1, wherein the IgG is IgG1, IgG2, IgG3 or IgG4.

3. The conjugate of claim 1, selected from the group consisting of Formulae 1-10:

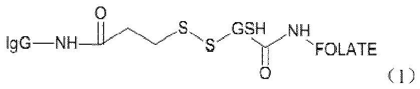

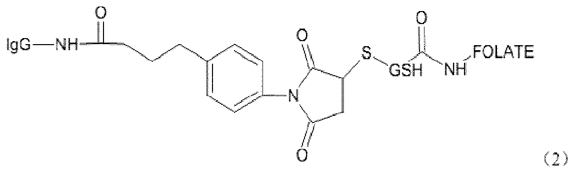

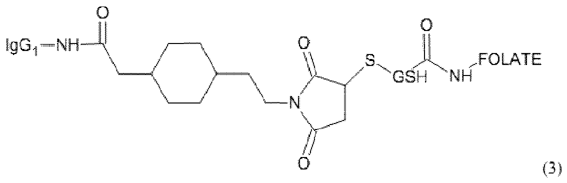

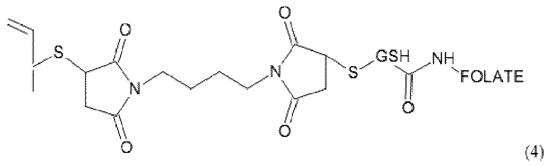

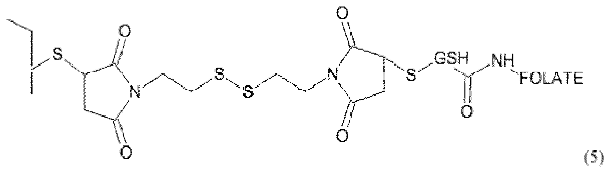

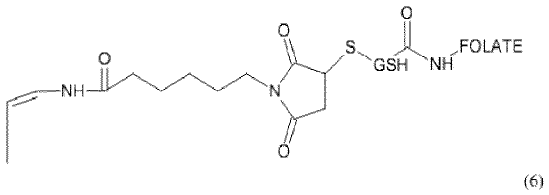

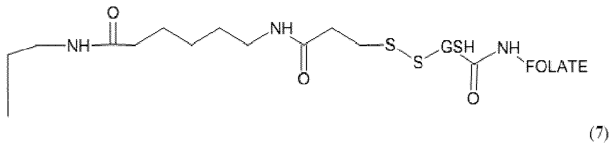

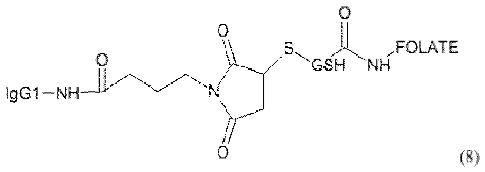

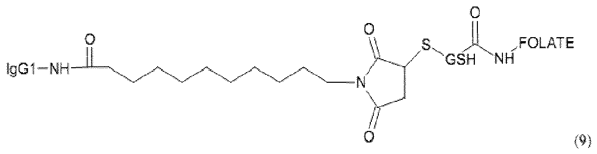

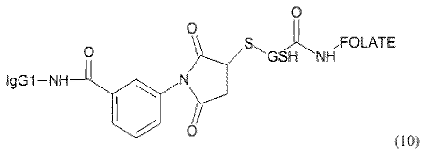

4. A method for synthesizing a conjugate of claim 1, comprising:
1) Coupling folate with N-hydroxysuccinimide to yield an active NHS-folate;
2) Coupling NHS-folate with GSH to yield folate-GSH through amide linkage;
3) Activating an IgG to generate an activated derivative of formula VI or formula VII, or a sulfhydryl reactive derivative having a formula IX: 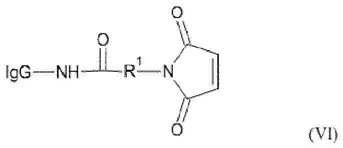 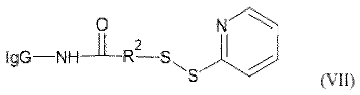 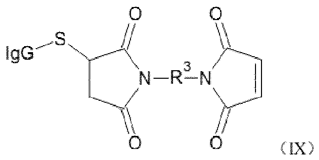 wherein:
R¹ is: 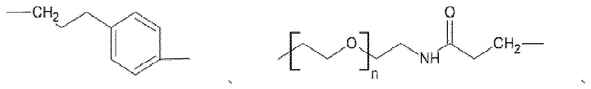 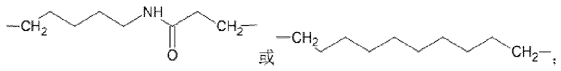 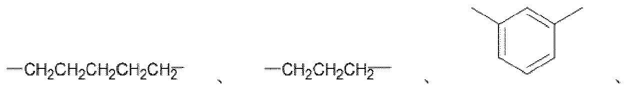 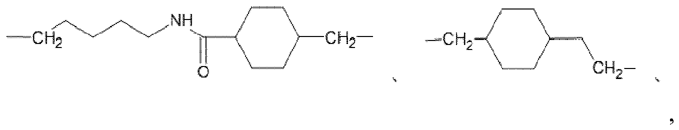
R² is 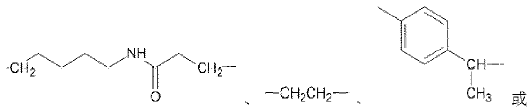 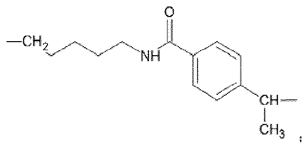
R³ is 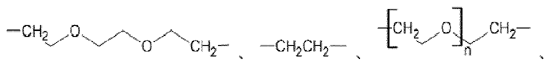 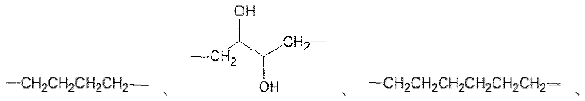 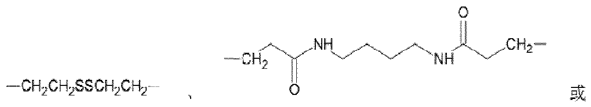 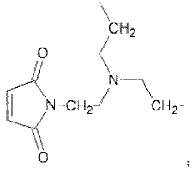
and
4) coupling the activation product IgG with a folate-GSH.

5. The method of claim 4, wherein folate is folate or a folate analogue selected from the group consisting of Methotrexate, Tetrahydrofolate and Dihydrofolate.

6. The method of claim 4, wherein the reaction conditions in step 1 are as follows: the folate or folate analogue dissolved in dimethylsulfoxide (DMSO) is reacted with 0.5-2 fold ofN, N '-dicyclohexylcarbodiimide and N-hydroxysuccinimide for 2-5 hrs, the insoluble byproduct is discarded through filtration or centrifugation, and NHS-activated folate (NHS-FOLATE) is in solution, wherein the the reaction equation is:

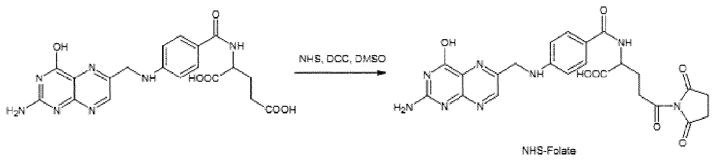

7. The method of claim 4, wherein in step 2, NSH-FOLATE is reacted with an equimolar amount of GSH overnight under argon in the dark, yielding FOLATE-GSH of the structure below:

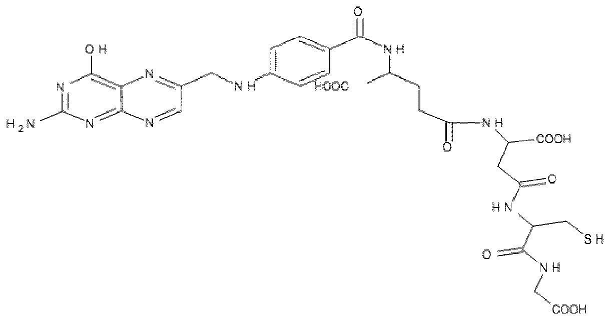

8. The method of claim 4, wherein the N-terminal amino group of the IgG is activated in accordance with the following reaction, 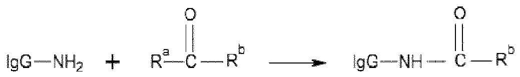 wherein R^{a}COR^{b} is a cross-linker in which R^{a} is a leaving group, and R^{b} contains a group which reacts with the free sulfhydryl in GSH,
wherein the cross-linker is Sulfo-LC-SPDP (sulfosuccinimidyl 6-[3' (2-pyridyldithio)-propionamido] hexanoate), LC-SPDP ((succinimidyl 6-[3' (2-pyridyldithio)-propionamido] hexanoate), SPDP (N-succinimidyl 3-(2-pyridyldithio) propionate), SMPT (4-Succinimidyloxycarbonyl-methyl-a-[2-pyridyldithio]toluene), Sulfo-LC-SMPT (4-Sulfosuccinimidyl-6-methyl-a-(2-pyridyldithio) toluamido hexanoate), Sulfo-SMCC (Sulfosuccinimidyl 4- [N-maleimidomethyl] cyclohexane-1-carboxylate), SM(PEG)n, NHS-PEG-Maleimide Crosslinker, SMCC (Succinimidyl 4-[N-maleimidomethyl]cyclohexane-1-carboxylate), LC-SMCC (Succinimidyl-4-[N-Maleimidomethyl]cyclohexane-1-carboxy-[6-amidocaproate]), Sulfo-EMCS ([N-epsilon-Maleimidocaproyloxy]sulfosuccinimide ester), EMCS ([N-epsilon- Maleimidocaproyloxy]succinimide ester), Sulfo-GMBS (N-[gamma-Maleimidobutyryloxy]sulfosuccinimide ester), GMBS (N-[gamma-Maleimidobutyryloxy]succinimide ester), Sulfo-KMUS (N-[kappa-Maleimidoundecanoyloxy]sulfosuccinimide ester), Sulfo-MBS (m-Maleimidobenzoyl-N-hydroxysulfosuccinimide ester), MBS (m-Maleimidobenzoyl-N-hydroxysuccinimide ester), Sulfo-SMPB (Sulfosuccinimidyl 4-[p-maleimidophenyl]butyrate), SMPB (Succinimidyl 4-[p-maleimidophenyl]butyrate), AMAS N-(alpha-Maleimidoacetoxy) succinimide ester), BMPS (N-[ß -Maleimidopropyloxy]succinimide ester), or SMPH (Succinimidyl-6-[ß-maleimidopropionamido] hexanoate), or group that can react with a free -SH group to yield an IgG with activated amino groups as shown in formula VI or VII: 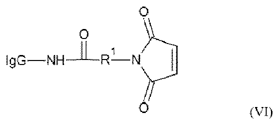 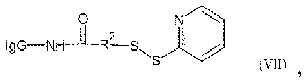 wherein R1 is 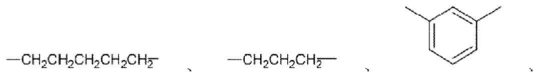 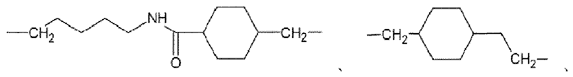 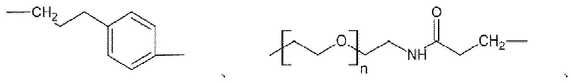 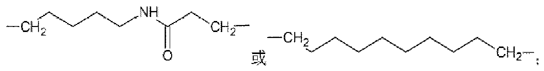 and R2 is

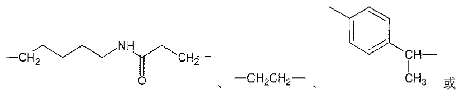

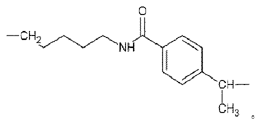

9. The method of claim 4, wherein in step 3 IgG is treated with a reducing agent to produce a half-IgG or a heavy chain, which is activated with a sulfhydryl linker , wherein the sulfhydryl linker is 1,8-bis-Maleimidodiethyleneglycol (BM(PEG)₂), BM(PEG)ₙ PEG Crosslinker, 1,4-bis-maleimidobutane (BMB), 1,4 bismaleimidyl-2,3-dihydroxybutane (BMDB), bismaleimidohexane (BMH), Bis-Maleimidoethane (BMOE), 1,4-Di-[3 '-(2'-pyridyldithio)-propionamido]butane (DPDPB), Dithio-bismaleimidoethane (DTME), Tris[2-maleimidoethyl]amine (TMEA), and an analogue thereof, to produce a conjugate of formula IX: 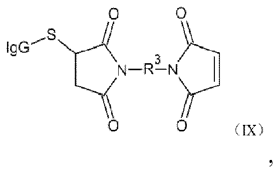 wherein R³ is:

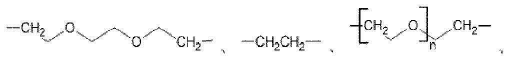

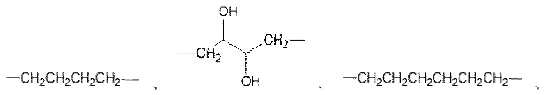

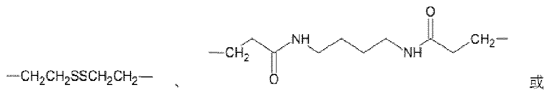

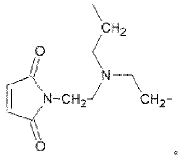

10. The method of claim 9, wherein IgG is activated to a half-IgG by 2-Mercaptoethylamine HCl (2-MEA) in accordance with the following reaction:

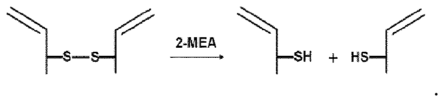

11. The method of claim 9, wherein in step 3 the IgG is reduced to an IgG heavy chain with 1,4-Dithiothreitol (DTT) in accordance with the following reaction:

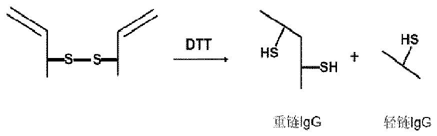

12. The method of any claim of claim 8-11, wherein in step 3, a solution of IgG with a concentration of 0.05 - 1mmol/L is obtained, and which is reacted with 10-30- fold of activated crosslinker for 0.5-1 h at room temperature or 1-3 h at 4 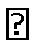 to obtain activated IgG.

13. The method of claim 12, wherein the activated IgG is purified with molecular sieve chromatography to relatively high purity.

14. The method of claim 12, wherein the IgG solution is in a solvent selected from 10 - 30mmol/L PBS-EDTA (pH6.5 ∼ 9.0), ultra pure water, DMSO or DMF.

15. The method of claim 4, wherein in step 4 FOLATE -GSH is reacted with 1-10-fold activated IgG for 2-5 h at room temperature or 15-20 h at 4 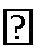 , to obtain a FOLATE-GSH-IgG conjugate of formula I-III.

16. The method of claim 15, wherein the purified conjugates are collected through molecular sieve chromatography.

17. The method of claim 4, wherein step 3 is performed prior to or simultaneously with steps 1 and 2.

18. A pharmaceutical composition comprising an effective amount of a folate-GSH-IgG conjugate of claim 1, and a pharmaceutically acceptable carrier or excipient.

19. Use of a folate-GSH-IgG conjugate of claim 1 for the treatment of a cancer or an autoimmune disease.

20. Use of claim 19, wherein the cancer is characterized with cancer cells having a high expression level of folate receptor.

21. Use of claim 20, wherein the cancer is leukemia, or an ovarian, cervical, endometrial, breast, colon, lung, liver and choroid cancer, or ependymoma.

22. Use of claim 19, wherein the autoimmune disease is characterized with diseased cells having a high expression level of folate receptor.

23. Use of Claim 23, wherein the autoimmune disease is rheumatoid arthritis, inflammatory bowel disease, psoriasis, lupus erythematosus, pulmonary fibrosis or sarcoidosis.

24. The use of claim 19, wherein the FOLATE-GSH-IgG conjugates is administered by injection.

25. The use of claim 24, wherein the FOLATE-GSH-IgG is administered by injection into the skin, muscles or veins.

26. The use of claim 19, wherein an effective dose of FOLATE-GSH-IgG conjugates in human is 0.1-4 g/kg (body weight).

27. The use of claim 26, wherein the effective dose of FOLATE-GSH-IgG conjugates in human is 0.1-1.0 g/kg (body weight).
